# EUROPÄISCHE PATENTSCHRIFT

(11) **EP 3 478 062 B1**
(45) Veröffentlichungstag und Bekanntmachung des Hinweises auf die Patenterteilung: **28.04.2021**
(21) Anmeldenummer: 17734095.7
(22) Anmeldetag: 30.06.2017
(51) Int. Cl.: A01N 25/08, A61L 2/20, A61L 11/00, B65D 85/34, B65F 7/00

(54) **VERWENDUNG VON ORGANISCHEN OZONIDEN IN BEHÄLTERN ZU DESINFEKTIONSZWECKEN**
USE OF ORGANIC OZONIDES IN CONTAINERS FOR DISINFECTING PURPOSES
UTILISATION D'OZONURES ORGANIQUES DANS DES RÉCIPIENTS À DES FINS DE DÉSINFECTION

(30) Priorität: 30.06.2016 DE 102016007928
(43) Veröffentlichungstag der Anmeldung: 08.05.2019
(73) Patentinhaber: Nonnenmacher, Klaus, 72070 Tübingen (DE)
(72) Erfinder: Nonnenmacher, Klaus, 72070 Tübingen (DE)
(74) Vertreter: Patentanwaltskanzlei Cartagena
(86) Internationale Anmeldenummer: PCT/EP2017/066234
(87) Internationale Veröffentlichungsnummer: WO 2018/002284

(56) Entgegenhaltungen:
- EP-A1- 2 179 748
- DE-A1-102011 013 920
- US-A- 2 512 640
- US-A1- 2003 044 314
- US-B1- 8 910 821

## Beschreibung

Die vorliegende Erfindung betrifft die Verwendung einer Einrichtung zur Anbringung in einem Behälter sowie eine Trägers, der von der Einrichtung umfasst ist, ein Verfahren für eine Behandlung von Material zu Desinfektionszwecken sowie einen Behälter, der mit dem Träger ausgestattet ist.

In nahezu allen Bereichen in Haushalt, Gewerbe und Industrie fallen ständig Abfälle an. Neben Kunststoffen und anderen Materialien enthalten die Abfälle in der Regel einen großen Anteil an organischem Material. In der Regel ist eine Mülltrennung vorgesehen, um kompostierbare Anteile zu separieren. Lose oder in Kunststofftüten, Papiertüten oder Zeitungspapier verpackt, werden die verschiedenen Müllanteile in Restmüllbehälter, Biomüllbehälter, Papierbehälter oder separat für ein Kunststoffrecycling zur Abholung bereitgestellt. Hierbei lagert der Abfallje nach Abholzyklus bis zu zwei Wochen oder länger in dem jeweiligen Müllbehälter. Insbesondere in den Sommermonaten, in denen die Abfälle höheren Temperaturen ausgesetzt sind, kann es zu erheblichen Insekten-, Bakterien-, Viren- und/oder Pilzentwicklungen in den Müllbehältern kommen. Je nach der im Müllbehälter herrschenden Temperatur können sich die hygienischen Verhältnisse darin dramatisch verschlechtern, sodass sich beispielsweise Bakterien oder Viren mit einer Verdopplungsrate pro halbe Stunde entwickeln können. Derartige Verhältnisse sind in der Regel mit einer erheblichen Geruchsentwicklung und Geruchsbelästigung verbunden. Auch die Entwicklung eines Madenbefalls oder die massenhafte Vermehrung von Fruchtfliegen ist sehr unangenehm und stellt ein nicht unerhebliches hygienisches Problem dar.

Zur Verbesserung der Hygiene gibt es bereits verschiedene Vorschläge. Beispielsweise wird empfohlen, gelöschten Kalk (Ca(OH)₂) in den Abfallbehälter zu geben. Der gelöschte Kalk wirkt als desinfizierendes Fungizid, ist jedoch stark basisch, sodass beispielsweise bei einem Kontakt mit den Augen die Gefahr von nicht reversiblen Augenschäden besteht. Ungelöschter Kalk (CaO) wird in Plumpsklos verwendet. Die Verwendung in Müllbehältern wäre brandgefährlich, da sich bei Kontakt mit Feuchtigkeit enorme Hitze entwickelt. Das Hypochlorid Chlorkalk (CaCl(OCl)) ist umweltgefährdend, da bei einer Umsetzung dieser Verbindung organische Chlorverbindungen (AOX) entstehen, die biologisch nicht abbaubar sind. Bei der Verwendung dieser Substanz zur Abfallbehandlung würden Deponien ihre Kompostierbarkeit verlieren, Verbrennungsanlagen Dioxine emittieren und Kläranlagen die AOX-Substanzen in Flüsse, Seen und Ozeane verfrachten. Insgesamt ist das Kalken von organischem Müll eine Umweltgefahr und birgt große Sicherheitsrisiken.

Die deutsche Patentschrift DE 197 28 609 C2 offenbart die Herstellung von Triglycerid-Sorbinsäure-Pantothenyl-Acetalperoxiden durch Behandlung einer heterogenen Mischung von flüssigen ungesättigten Triglyceriden (Pflanzenölen), flüssigem Pantothenyl-Alkolhol, festen feinteiligen oder flüssigen Polyethylenglykolen und fester feinteiliger Sorbinsäure mit ozonhaltigem Sauerstoff, wobei die hergestellte Substanz in Form einer wässrigen Emulsion zur Bekämpfung von pathogenen Pilzen oder Bakterien bei einer äußerlichen oder innerlichen Anwendung beim Menschen vorgesehen ist. Die deutsche Offenlegungsschrift DE 199 32 570 A1 offenbart die Verwendung dieser Substanz, die als mit Ozon angereichertes Oliven- und Rizinusöl beschrieben wird, für eine antimikrobielle Behandlung im Zahnbereich, wobei in Bezug auf die Herstellung dieser Substanz auf die zuvor genannten Patentschrift verwiesen wird. Die Substanz wird direkt auf oder im Zahnfleisch appliziert.

Eine medizinische Hautabdeckung zur Behandlung von Infektionen der Haut ist aus der DE 102011013920 A1 bekannt. Das Pflaster umfasst Ozonide und/oder Hydroperoxide in Form eines ozonisierten Pflanzenöls.

Aus der US 8910821 B1 ist ein Müllbehälter bekannt, welcher eine Aufnahme für ein Desinfektionsmittel, insbesondere eines festen Desinfektionsmittels, aufweist. Das Desinfektionsmittel soll insbesondere im Inneren des Müllbehälters Wirkung entfalten. Es ist nicht spezifiziert, welche Art von Desinfizierungsmittel in dem beschriebenen Müllbehälter zum Einsatz kommen kann.

Aus der US 2003/044314 A1 ist ein Papierprodukt bekannt, das mit einer Mischung aus Wasserstoffperoxid und Essigsäure behandelt wurde. In Folge einer chemischen Reaktion der beiden Reagenzien umfasst es auch ein organisches Hydroperoxid, nämlich Peroxyessigsäure. Das Papierprodukt findet Verwendung zum Sterilisieren von Oberflächen.

Aus der EP 2179748 A1 ist ein geruchsoptimierendes Produkt bekannt, das u.a. in einem Müllbehälter angeordnet werden kann. Das Produkt umfasst einen Feststoff, der mit einem Persauerstoffbleichmittel versetzt ist. Dabei kann es sich um ein Alkylhydroperoxid handeln.

In Bezug auf die Problematik der hygienischen Verhältnisse bei der Abfalllagerung oder allgemein in Bezug auf die Zwischenlagerung von Material stellt sich die Erfindung die Aufgabe, einen besseren Ansatz im Zusammenhang mit der Zwischenlagerung von Material, insbesondere von organischem Material, bereitzustellen, wobei der Ansatz umweltschonend sein soll und zugleich die hygienischen Verhältnisse bei der Zwischenlagerung von Abfällen verbessern soll. Diese Aufgabe wird durch die Verwendungen gemäß den Ansprüchen 1 und 9, das Verfahren gemäß Anspruch 10 und den Behälter gemäß Anspruch 14 gelöst.

Die Erfindung stellt für die erfindungsgemäße Verwendung eine Einrichtung zur Anbringung in einem Behälter, der für eine Zwischenlagerung von Material vorgesehen ist, bereit, der eine erhebliche Verbesserung der hygienischen Verhältnisse in dem Behälter ermöglicht. Bei dem Behälter handelt es sich bevorzugt um einen Mülleimer, wie er in Privathaushalten zum Einsatz kommt.

Erfindungsgemäß umfasst die Einrichtung wenigstens einen Träger, der mit organischen Ozoniden oder einer Mischung aus organischen Ozoniden und organischen Hydroperoxiden versetzt ist. Anders als bei der herkömmlichen Verwendung von ozonbehandelten Pflanzenölen (Ozoniden), beispielsweise bei der Behandlung im Mundraum, wird bei der vorliegenden Erfindung das organische Ozonid oder die Mischung aus dem organischen Ozonid und dem Hydroperoxid nicht direkt auf das zu behandelnde Material aufgebracht. Vielmehr basiert die Wirkung, die mit der bereitgestellten Einrichtung erreicht wird, auf den sich entwickelnden Gasen oder Dämpfen, die sich in der Masse des organischen Ozonids oder der Mischung aus dem organischen Ozonid und dem Hydroperoxid (im Folgenden auch als Ozonid-/Hydroperoxidmasse bezeichnet) entwickeln. Von dem zu behandelnden Material selbst, beispielsweise organischem Material, wird insbesondere bei wärmeren Temperaturen Wasserdampf freigesetzt, wobei durch Kondensation auch flüssiges Wasser auftreten kann. Bei Kontakt der gasförmigen Phase der organischen Ozonid- oder der Ozonid-/Hydroperoxidmasse selbst mit dieser Feuchtigkeit im Behälter bildet sich vor allem Wasserstoffperoxid, welches oxidierend und desinfizierend wirkt. Etwaige Carbonsäuren, die bei diesen Vorgängen als Abbauprodukte ebenfalls gebildet werden, können die Kondensation begünstigen und die Prozesse beschleunigen. Die hierbei entstehende desinfizierende Atmosphäre verhindert die Entwicklung von Bakterien, Pilzen, Viren und/oder Insekten zuverlässig oder verlangsamt sie zumindest deutlich.

Bei den organischen Ozoniden oder organischen Hydroperoxiden handelt es sich um Substanzen, die durch eine Behandlung von ungesättigten Fettsäuren (Fettsäuren mit CC-Doppelbindungen) mit ozonhaltigem Sauerstoff hergestellt wurden. Für die Herstellung der Ozonide und Peroxide wird dabei zweckmäßigerweise gasförmiger und mit Ozon angereicherter Sauerstoff eingesetzt, der vorzugsweise in eine Reaktionsmischung mit den Fettsäuren bei intensiver Durchmischung eingeleitet wird. Bei der Herstellung ist es prinzipiell möglich, feuchten oder trockenen ozonhaltigen gasförmigen Sauerstoff zu verwenden. Bei der Verwendung von feuchtem ozonhaltigem Sauerstoff entstehen in erster Linie organische Hydroperoxide. Bei der Verwendung von trockenem ozonhaltigem Sauerstoff entstehen in erster Linie organische Ozonide, die monomer oder polymer sein können. Hierbei reagiert das Ozon spontan an der CC-Doppelbindung und bildet ein beständiges Ozonid. Sofern die die Fettsäuren enthaltene Reaktionsmischung Wasser enthält, können sich bei der Reaktion mit dem Ozon des trockenen Sauerstoffs ebenfalls organische Hydroperoxide bilden.

Zur Klarstellung: Das Wasser in der Reaktionsmischung (unabhängig davon, ob es direkt oder in Form von Feuchtigkeit dem mit Ozon angereicherten Sauerstoff zugegeben wird) dient nicht etwa zur Verdünnung, es ist vielmehr Reaktionspartner und wird im Laufe der Ozonisierung verbraucht. Wenn der Reaktionsmischung Wasser unterstöchiometrisch zugesetzt wird (es werden weniger mol Wasser zugesetzt als CC-Doppelbindungen vorhanden sind), so erhält man nach Abschluss der Ozonisierung ein trockenes Ozonisierungsprodukt.

Entsprechend ist in besonders bevorzugten Ausführungsformen der Anteil an zugesetztem Wasser auch stöchiometrisch auf die Menge der in den Fettsäuren enthaltenen Doppelbindungen abgestimmt. Demzufolge sollte idealerweise maximal 1 mol H2O/mol CC-Doppelbindungen zugesetzt werden.

Besonders bevorzugt werden den Fettsäuren je mol CC-Doppelbindungen zwischen 0,01 und 1 mol Wasser, bevorzugt von 0,1 mol bis 0,9 mol Wasser, weiter bevorzugt von 0,1 mol bis 0,75 mol Wasser, besonders bevorzugt von 0,1 mol bis 0,7 mol Wasser, zugesetzt. Innerhalb der genannten Bereiche sind Wasseranteile von 0,3 mol bis 0,9 mol, bevorzugt von 0,3 mol bis 0,75 mol, insbesondere von 0,5 mol bis 0,75 mol Wasser je mol CC-Doppelbindungen für einige Anwendungen weiter bevorzugt.

In besonders bevorzugter Weise wird reine Ölsäure als Fettsäure für die Herstellung der organischen Ozonide oder der Mischung aus den organischen Ozoniden und den organischen Hydroperoxiden verwendet, da sich die hieraus herstellbaren Substanzen als besonders effektiv für die erfindungsgemäßen Zwecke erwiesen haben. Es können aber auch alle ölsäurehaltige Mischungen verwendet werden, die mehr als 50 Gew.-% an Ölsäure enthalten. Im Grunde ist jede ungesättigte Fettsäure einsetzbar, die dazu geeignet ist, mit Ozon ein Ozonid zu bilden.

In einer besonders bevorzugten Ausgestaltung der Einrichtung ist der wenigstens eine Träger mit organischen Ozoniden versetzt, die durch die Behandlung der ungesättigten Fettsäuren mit trockenem ozonhaltigem Sauerstoff herstellbar sind, wobei insgesamt der Herstellungsprozess der Ozonide vorzugsweise unter aprotischen Bedingungen durchgeführt wird. Gemäß dem von Rudolf Criegee beschriebenen Reaktionsmechanismus werden hierbei neben der Bildung von verschiedenen Zwitterionen ein Primärozonid (Molozonid) und ein Sekundärozonid (1,2,4-Trioxolan) gebildet (Lit.: Von Sonntag, Chemistry of ozone in water and waste water treatment, page 85, ISBN 9781843393139). Das bei dieser Reaktion letztendlich gebildete Sekundärozonid wird auch als Criegee-Ozonid bezeichnet und ist für die Zwecke der vorliegenden Erfindung besonders bevorzugt.

Die Verwendung der trocken bzw. aprotisch hergestellten organischen Ozonide für die Zwecke der Erfindung hat gegenüber der Verwendung von organischen Hydroperoxiden verschiedene Vorteile. So sind die weiter unten noch näher erläuterten Peroxidzahlen (POZ) von Ozoniden im allgemeinen höher als die Peroxidzahlen von Hydroperoxiden. Damit ist auch die Wirksamkeit der Ozonide in der Einrichtung höher. Die trocken hergestellte Ozonidform wartet dabei gewissermaßen dringlich auf Wasser, um mit diesem zum desinfizierend wirkenden Wasserstoffperoxid zu reagieren. Das feucht hergestellte organische Hydroperoxid hat sich das Wasser gewissermaßen schon einverleibt, sodass sich das Wasserstoffperoxid bereits in der Hydroperoxidsubstanz befindet und die Reaktivität mit Wasser weniger stark ausgeprägt ist. Hinzu kommt, dass die Lagerfähigkeit des organischen Hydroperoxids sehr begrenzt ist. Die trocken hergestellte und trocken gelagerte Ozonidform hingegen ist lange haltbar und stabil, wobei die Reaktivität erhalten bleibt. Wichtig hierbei ist, dass kein Wasser in die Ozonidsubstanz vor der Anwendung (also bevor der Träger mit der Ozonidsubstanz seine desinfizierende Wirkung entfalten soll) eindiffundiert, weshalb der Träger mit der Ozonidsubstanz vor seiner Anwendung entsprechend wasserdiffusionsdicht verpackt sein sollte.

Als Transport- und/oder Lagerbehältnisse für den Träger mit den organischen Ozoniden oder der Mischung aus den organischen Ozoniden und den organischen Hydroperoxiden eignen sich insbesondere Behälter aus Glas oder aus wasserdichten Metall- und/oder Kunststofffolien, insbesondere wasserdiffusionsdichten Verbundfolien. Geeignete Verbundfolien weisen beispielsweise eine dünne Lage aus einem Metall als Diffusionssperre auf.

Zweckmäßigerweise weist das verwendete organische Ozonid oder die Mischung aus dem organischen Ozonid und dem organischen Hydroperoxid einen relativ hohen Dampfdruck auf, da bei dem erfindungsgemäßen Ansatz insbesondere auch die sich aus der Ozonid-/Hydroperoxidmasse entwickelnde Gasphase bzw. die sich entwickelnden Dämpfe die desinfizierende Wirkung entfalten. Diese Dämpfe können im Prinzip alle Bereiche des Abfalls bzw. der organischen Materials durch Diffusion durchdringen und auch in die Materialien selbst eindringen. Zusammen mit dem Wasserdampf, der aus dem zu behandelnden Material entwickelt wird, kommt es zur Ausbildung von desinfizierend wirkendem Wasserstoffperoxid, so dass sich eine desinfizierende Atmosphäre einstellt. Allgemein setzt sich der in einem geschlossenen Behälter herrschende Gesamtdruck aus den einzelnen Partialdrücken der in dem Inneren des Behälters vorhandenen Komponenten zusammen, beispielsweise dem Partialdruck des Sauerstoffs, dem Partialdruck des Stickstoffs, dem Partialdruck von vorhandenem Wassergas, dem Partialdruck der Ozonide oder der Mischung aus den organischen Ozoniden und den Hydroperoxiden und dem Partialdruck von gegebenenfalls vorhandenen weiteren Komponenten. Wenn beispielsweise in eine luftgefüllte offene Flasche ein mit Wasser getränktes Vliespapier eingelegt wird und die Flasche mit einem Stopfen verschlossen wird, in welchem ein mit Wasser gefülltes U-Rohr als Manometer eingebracht ist, ist ein in der Flasche entstehender Druck in mm Wassersäule ablesbar. Nach einer bestimmten Zeit ist ein Gleichgewichtszustand bzw. ein Gleichgewichtsdruck erreicht. Die Erhöhung des Drucks in der Flasche entspricht dabei dem Sattdampfdruck des Wassers aus dem Vliespapier. Der Sattdampfdruck (Sättigungsdampfdruck) ist dabei eine stoffspezifische Größe. Während des Druckanstiegs geht dieses Wasser durch Verdunstung in die trockene Luft über. Die treibende Kraft für diesen Stoffübergang ist der Unterschied der Wasserdampfpartialdrücke des nassen Papiers und des Wasserdampfdruckes in der Luft innerhalb der Flasche. Wenn der Wasserdampfdruck in der Flasche gleich dem Sättigungsdruck (Sattdampfdruck) ist, ist das treibende Gefälle Null und die weitere Verdunstung des Wassers hört auf. Als Modell für den erfindungsgemäßen Ansatz wird in die Flasche zusätzlich zu dem mit Wasser getränkten Vliespapier ein mit einem Ozonid getränktes Papier zugegeben. Hierbei erhöht sich der Sattdampfdruck des Wassers um den Sattdampfdruck des Ozonids. Es kommt nun zu einer Kondensation des Wassers, da dessen Sättigungsdruck überschritten ist. Hierbei kommen die Ozoniddämpfe mit dem Wasser in Kontakt und es bildet sich Wasserstoffperoxid, so dass sich eine desinfizierende Atmosphäre in der Flasche ausbildet. Die Erhöhung des Gesamtdrucks in der Flasche bzw. in dem Behälter durch das in den Behälter eingebrachte Ozonid oder die Mischung aus dem organischen Ozonid und dem Hydroperoxid ist ein wichtiger physikalischer Effekt, der die Aerosolbildung und damit die Wirkung des Wasserstoffperoxids gemäß dem erfindungsgemäßen Ansatz verursacht.

Der Dampfdruck der Ozonid-/Hydroperoxidmasse hängt unter anderem von der Peroxidzahl (POZ) der jeweiligen Masse ab. Die POZ stellt dabei eine Kennzahl für den Gehalt an peroxidischen bzw. reaktiven Sauerstoffgruppen der jeweiligen Substanz (Fett oder Öl) dar. Die POZ wird gemäß DIN-Normen mittels Titration, beispielsweise mittels Iodometrie gemäß DIN EN ISO 3960:2017, bestimmt und wird in "Milli-Äquivalent Sauerstoff je Kilogramm Substanz (meq/kg)" angegeben. Für die Zwecke der Erfindung werden vorzugsweise organische Ozonide oder Mischungen aus den organischen Ozoniden und den organischen Hydroperoxiden mit POZ in einem Bereich zwischen etwa 50 - 5000 meq/kg, vorzugsweise zwischen etwa 100 - 2500 meq/kg, eingesetzt. Besonders geeignet sind organische Ozonide mit POZ zwischen 1000 - 2500 meq/kg.

Die POZ lässt sich durch die Bedingungen bei der Ozonid- und Hydroperoxid-Herstellung steuern. Beispielsweise erhöht die Reinheit bzw. Qualität des verwendeten ozonhaltigen Sauerstoffgases die POZ des gebildeten Produktes. Weiterhin besteht ein logarithmischer Zusammenhang zwischen der Dauer der Ozonisierung und dem POZ-Wert des ozonidhaltigen Produktes, wobei die POZ mit zunehmender Dauer ansteigt und je nach Reaktionsbedingungen beispielsweise bei 6 h eine Sättigung zeigt. In der Praxis hat sich beispielsweise eine Ozonisierungsdauer von 4 h bei einem Batch-Verfahren bewährt.

Insbesondere ist es auch durch den Zusatz von Wasser zu der oben erwähnten Reaktionsmischung möglich, das Verhältnis der Menge entstehenden Ozonids zur Menge entstehenden Hydroperoxids und damit auch die Peroxidzahl (POZ) gezielt zu beeinflussen. Grundsätzlich gilt, dass ein geringerer Wasserzusatz in der Reaktionsmischung mit einem höheren Anteil an entstehendem Ozonid verknüpft ist. Und je höher der Anteil des entstehenden Ozonids ist, desto höher ist in der Regel auch die POZ.

So wird zur Einstellung der POZ der organischen Ozonide oder der Mischung aus den organischen Ozoniden und den organischen Hydroperoxiden auf den genannten bevorzugten Bereich zwischen 1000 und 2500 meq/kg der Wasserzusatz in der genannten Reaktionsmischung in der Regel auf einen Wert innerhalb eines der genannten Molbereiche eingestellt.

Grundsätzlich wird mit zunehmender POZ die Viskosität der Substanz größer, wobei die Viskosität beispielsweise durch die Zugabe von Lösungsmitteln, z.B. Polyethylenglykol, beeinflusst werden kann. Eine Erniedrigung der Viskosität durch Zusatz eines Lösungsmittels kann beispielsweise vorteilhaft sein, um die Benetzbarkeit des Trägers der Einrichtung mit der Ozonid-/Hydroperoxidmasse zu verbessern.

Mit der bereitgestellten Einrichtung wird das organische Ozonid oder die Mischung aus dem organischen Ozonid und dem Hydroperoxid in das Innere des Behälters mittels des Trägers eingebracht. Vorzugsweise umfasst die Einrichtung eine Halterung für den Träger, wobei die Halterung im inneren Bereich des Behälters anbringbar ist. Prinzipiell ist es auch möglich, den Träger von außen anzubringen, sofern gewährleistet ist, dass durch geeignete Löcher oder Aussparungen in der Behälterwandung die sich entwickelnden Dämpfe in das Innere des Behälters gelangen. In besonders bevorzugter Weise kann die Halterung an der Innenseite eines Deckels des Behälters anbringbar sein. Die Halterung kann auf verschiedene Weise im Behälter befestigt werden, beispielsweise durch Schraubung oder Klebung. Auch die Befestigung des Trägers in der Halterung kann auf verschiedene Weise erfolgen. Beispielsweise kann die Halterung so eingerichtet sein, dass der Träger mit dem Ozonid oder der Mischung aus dem organischen Ozonid und dem Hydroperoxid eingeklemmt oder eingeschoben wird. Auch eine Befestigung mittels Nadeln, Klettverschluss oder Klebung ist möglich. In einer besonders einfachen Ausgestaltung der Einrichtung kann als Halterung ein Loch beispielsweise im Deckel des Behälters vorgesehen sein, in das der Träger mit dem Ozonid oder der Mischung aus dem organischen Ozonid und dem Hydroperoxid eingesteckt und dabei eingeklemmt wird, sodass der Träger in das Innere des Behälters hineinragt. Bei hohen Temperaturen und insbesondere bei direkter Sonneneinstrahlung auf den Behälter ist die Verdunstungsrate im Behälter besonders hoch. Der sich bildende Wasserdampf steigt im Behälter nach oben und das kondensierende Wasser sammelt sich an der Unterseite des Behälterdeckels. Es ist daher besonders vorteilhaft, wenn die Einrichtung an der Innenseite des Deckels angebracht ist, da an dieser Stelle der aufsteigende Wasserdampf und/oder das kondensierte Wasser in zuverlässiger Weise in direkten Kontakt mit dem Träger für das Ozonid oder die Mischung aus dem Ozonid und dem Hydroperoxid in Kontakt tritt und sich die desinfizierende Atmosphäre in besonders effektiver Weise ausbilden kann.

In besonders bevorzugter Weise ist der Träger mit dem organischen Ozonid oder der Mischung aus dem organischen Ozonid und dem Hydroperoxid austauschbar, sodass ein neuer Träger mit frischem Ozonid und gegebenenfalls Hydroperoxid eingesetzt werden kann, wenn das Ozonid oder die Mischung aus dem Ozonid und dem Hydroperoxid des vorherigen Trägers gewissermaßen verbraucht ist. Es ist möglich, dass der Träger zusammen mit dem Material im Behälter entsorgt wird, wenn das Material beispielsweise von der Müllabfuhr abgeholt wird. Hierfür kann in Zuge des Bereitstellens des Behälters für die Abholung der Träger mit dem Ozonid oder der Mischung aus dem Ozonid und dem Hydroperoxid aus seiner Halterung entnommen und dem Material zugegeben werden. Nach dem Entleeren des Behälters wird ein neuer Träger mit frischer Ozonid-/Hydroperoxidmasse in der Halterung befestigt.

Vorzugsweise umfasst der Träger ein poröses Material. Dies hat den Vorteil, dass das organische Ozonid oder die Mischung aus dem organischen Ozonid und dem Hydroperoxid zum Teil in den Träger eindringen kann und gut daran haftet. Da vor allem das organische Ozonid im Wesentlichen wasserfrei und hydrophob ist, kann durch eine gewisse Porosität die Aufnahmekapazität des Trägers für das organische Ozonid verbessert werden, auch im Hinblick auf einen Transport und eine Verpackung des mit dem organischen Ozonid versehenen Trägers. Durch eine Porosität des Trägers wird weiterhin die Oberfläche des Trägers vergrößert. Neben einer verbesserten Adhäsion des organischen Ozonids oder der Mischung aus dem organischen Ozonid und dem Hydroperoxid auf dieser Oberfläche wird damit auch die Verdampfungsrate erhöht. Da bei dem erfindungsgemäßen Ansatz die sich entwickelnden Dämpfe der Ozonid-/Hydroperoxidmasse die desinfizierende Wirkung entfalten, wird daher durch eine entsprechende Porosität des Trägers die Wirkung verstärkt. Vorzugsweise weist der Träger zumindest teilweise eine adhäsive Oberfläche auf, sodass das Ozonid oder die Mischung aus dem Ozonid und dem Hydroperoxid gut anhaften kann. Allgemein eignen sich für den Träger poröse und/oder saugfähige Materialien wie z. B. vliesartige Gewebe aus Baumwolle oder Ähnlichem. Weiterhin eignen sich Trägermaterialien mit einer vergrößerten Oberfläche, an denen das organische Ozonid oder die Mischung aus dem Ozonid und dem Hydroperoxid anhaftet und/oder in die die Substanz zumindest teilweise eindringen kann, beispielsweise Holz, Zeolithe, Keramik, Glas, Watte, Filz oder ähnliche Textilfasern, Aktivkohle oder geschäumte oder faserige Kunststoffe, wie beispielsweise Polyethylen. Bei der Verwendung von Holz ist es vorteilhaft, dass der Schnitt des Holzes so erfolgt, dass die Richtung der Kapillaren innerhalb des Holzes der Diffusionsrichtung entspricht, sodass die Dämpfe durch das Holz in das Innere des Behälters entweichen können. Weiterhin können auch metallische Drahtgewebe als Träger eingesetzt werden, die vorzugsweise eine aufgeraute metallische Oberfläche aufweisen. Derartige Drahtgewebe, die mit dem organischen Ozonid oder der Mischung aus dem organischen Ozonid und dem Hydroperoxid versetzt werden, können beispielsweise rollenartig aufgewickelt oder Z-förmig gefaltet sein. Zweckmäßigerweise wird das Material des Trägers so gewählt, dass es mit dem Ozonid oder der Mischung aus dem organischen Ozonid und dem Hydroperoxid und dem sich im Zuge derAnwendung entwickelnden Wasserstoffperoxid im Wesentlichen nicht reagiert.

In einer besonders bevorzugten Ausgestaltung der Einrichtung besteht der Träger im Wesentlichen aus einem biologisch abbaubarem Material. Dies hat den besonderen Vorteil, dass dann der Träger zusammen mit dem zu behandelnden Material einer Kompostierung zugeführt werden kann. Für diesen Aspekt der Erfindung eignen sich beispielsweise Materialien auf der Basis von Zellulose oder andere bakteriologisch abbaubare Materialien.

Je nach den Eigenschaften des Trägermaterials wird der Träger mit dem organischen Ozonid oder der Mischung aus dem organischen Ozonid und dem Hydroperoxid, das selbst eine ölige bis pastöse Konsistenz aufweist, bestrichen und/oder getränkt. Vor Vorteil ist es, wenn das Trägermaterial eine hohe Aufnahmekapazität, insbesondere durch eine entsprechende Porosität oder Saugfähigkeit, aufweist, sodass die Substanzmasse des gebrauchsfertigen Trägers ausreichend groß ist, um die desinfizierende Wirkung über einen längeren Zeitraum zu gewährleisten.

Da die desinfizierende Wirkung des erfindungsgemäßen Ansatzes auf der Entwicklung von Dämpfen aus der Ozonid- und gegebenenfalls der Hydroperoxidmasse und von Wasserdämpfen im Inneren des Behälters beruht, sollte zweckmäßigerweise der Behälter während der Anwendung geschlossen sein. Vorzugsweise ist der Behälter für die Zwischenlagerung des organischen Materials daher mit einem Deckel versehen.

In einer weiteren bevorzugten Ausgestaltung der Einrichtung umfasst die Einrichtung einen Vorratstank für die Ozonid-/Hydroperoxidmasse. Hierbei kann es vorgesehen sein, dass der Vorratstank nachfüllbar ist und eine wiederverschließbare Füllöffnung aufweist, sodass "frische" Ozonid-/Hydroperoxidmasse vom Anwender bei Bedarf nachgefüllt werden kann. Der Träger, der in das Innere des Behälters hineinragt, steht in Kontakt mit dem Inneren des Vorratstanks, wobei die Ozonid-/Hydroperoxidmasse beispielsweise durch Kapillarkräfte in den Träger eingesogen werden kann. Hierfür kann der Träger beispielsweise auf einem Vliesmaterial basieren, das sich fortwährend mit der Ozonid-/Hydroperoxidmasse aus dem Vorratstank vollsaugt. Es kann auch vorgesehen sein, dass der Vorratstank für den Anwender nicht wiederbefüllbar ist und also keine zugängliche Füllöffnung aufweist. In dieser Ausgestaltung wird der Vorratstank einmalig befüllt und dann für die Anwendung bereitgestellt. Eine Einrichtung mit Vorratstank hat den Vorteil, dass die Einrichtung über einen längeren Zeitraum verwendet werden kann, da der Träger fortwährend mit "frischer" Ozonid-/Hydroperoxidmasse versorgt wird, bis die Masse aus dem Vorratstank verbraucht ist, wobei die Ozonid-/Hydroperoxidmasse dann gegebenenfalls nachgefüllt werden kann. Die Einrichtung mit einem Vorratstank kann beispielsweise in Form eines Steckknopfes ausgestaltet sein, der gegebenenfalls zusammen mit einer geeigneten Dichtung in eine Öffnung eines herkömmlichen Abfallbehälters, beispielsweise in eine Bohrung in der Behälterwandung (Korpus) oder in dem Behälterdeckel, eingesteckt wird.

Die Erfindung umfasst weiterhin die Verwendung eines Trägers, der mit organischen Ozoniden oder einer Mischung aus organischen Ozoniden und organischen Hydroperoxiden versetzt ist, zur Bereitstellung einer Atmosphäre mit desinfizierender Wirkung. Dieser Träger ist insbesondere zur Einbringung in einen Behälter, der für eine Zwischenlagerung von Material vorgesehen ist, beispielsweise einem Abfallbehälter, vorgesehen. Weiterhin ist der erfindungsgemäß verwendbare Träger beispielsweise für die Behandlung von Pflanzen im Sinne einer Schädlingsbekämpfung geeignet, wie weiter unten noch näher ausgeführt wird. Bezüglich weiterer Merkmale dieses Trägers wird auf die obige Beschreibung verwiesen.

Weiterhin umfasst die Erfindung einen mit einem Deckel versehenen, für eine Zwischenlagerung von Material vorgesehenen, verschließbaren Behälter, umfassend einen Träger, der mit organischen Ozoniden oder einer Mischung aus organischen Ozoniden und organischen Hydroperoxiden versetzt ist, die durch eine Behandlung von ungesättigten Fettsäuren mit ozonhaltigem Sauerstoff hergestellt wurden, und der in dem Behälter eine Atmosphäre mit desinfizierender Wirkung bereitstellt.

Schließlich umfasst die Erfindung ein Verfahren zur Behandlung von Material insbesondere zu Desinfektionszwecken bzw. zur Verbesserung der Hygiene. Allgemein eignet sich das erfindungsgemäße Verfahren zur Behandlung von organischem Material oder von sonstigem wasserhaltigem oder wasserproduzierendem Material. Die Wasserhaltigkeit oder die wasserproduzierenden Eigenschaften des zu behandelnden Materials sind vorteilhaft, da die Funktion des erfindungsgemäßen Ansatzes auch auf dem Zusammenspiel von sich entwickelnden Ozonid- und gegebenenfalls Hydroperoxiddämpfen und Wasser basiert. Gemäß dem erfindungsgemäßen Verfahren wird das Material in einem Behälter zwischengelagert, wobei in dem Behälter ein mit organischen Ozoniden oder einer Mischung aus organischen Ozoniden und organischen Hydroperoxiden versetzter Träger eingebracht ist oder wird. Eine desinfizierende Wirkung auf das organische Material wird dadurch erzielt, dass in das Innere des Behälters Dämpfe entweichen, die sich aus der Ozonid-/Hydroperoxidmasse, mit der der Träger versetzt ist, entwickeln. In Zusammenspiel mit Wasserdämpfen, die sich am organischen Material bilden, kommt es zu einer Bildung von Wasserstoffperoxid und damit zu einer desinfizierenden Wirkung, die beispielsweise die Entwicklung von Bakterien, Pilzen, Viren oder Insekten verhindert oder zumindest zu einem großen Teil verringert. Bezüglich weiterer Merkmale des Trägers und des mit Ozonid und gegebenenfalls zusätzlich mit Hydroperoxid versetzten Trägers wird auf die obige Beschreibung verwiesen.

Die wirksamen Konzentrationen, die für das erfindungsgemäße Verfahren eingesetzt werden können, lassen sich prinzipiell anhand der Verfärbung eines Kaliumiodid-Stärkepapiers messen, beispielsweise nach 4 Stunden Einwirkungszeit. Die Verfärbung des Kaliumiodid-Stärkepapiers spiegelt im Wesentlichen den Wasserstoffperoxid-Gehalt wider. Die Wirkung lässt sich allgemein als CT-Wert umschreiben, wobei sich der CT-Wert auf die Konzentration (mg/l) multipliziert mit der Zeit bezieht, also beispielsweise mg/l x Minute. Eine Konzentration von 0,01 mg H₂O₂/l verfärbt nach 4 Stunden bereits das Kaliumiodid-Stärkepapier. Der entsprechende CT-Wert ergibt sich daraus mit
CT = 0,01 mg H₂O₂/l x 240 min = 2,4

In besonders bevorzugter Weise eignet sich dieses Verfahren für die Behandlung von organischen Abfällen und kann beispielsweise in einem im Prinzip handelsüblichen Abfallbehälter eingesetzt werden. Die Erfindung kann im Außenbereich und im Innenbereich zum Einsatz kommen. Herkömmlicherweise kommt es vor allem in Abfallbehältern, die für kompostierbaren Abfall vorgesehen sind, zu einer erheblichen Entwicklung von Bakterien, Viren, Pilzen und/oder Insekten, was mit einer unangenehmen Geruchsentwicklung und unter Umständen auch mit gesundheitlichen Risiken verbunden ist. Das erfindungsgemäße Verfahren eignet sich in besonders effektiver und umweltschonender Weise dafür, die hygienischen Verhältnisse in einem derartigen Abfallbehälter zu verbessern. Hierbei stellt das erfindungsgemäße Verfahren und die oben beschriebene erfindungsgemäß verwendbare Einrichtung eine sehr kostengünstig und einfach zu realisierende Möglichkeit dar, die prinzipiell für eine flächendeckende Anwendung geeignet ist.

Darüber hinaus eignet sich das erfindungsgemäße Verfahren auch zur Behandlung von anderen organischen Materialien und insbesondere von anderen Abfällen. Beispielsweise kann das Verfahren für die Behandlung von Hygieneabfällen, beispielsweise zur Behandlung von gebrauchten Babywindeln oder Ähnlichem, eingesetzt werden. Dabei eignet sich das erfindungsgemäße Verfahren in besonderer Weise beispielsweise auch für die Zwischenlagerung von Krankenhausabfällen, die verschiedene organische Anteile, z.B. Blut und/oder Urin, enthalten können und die mit einer erheblichen Keimbelastung versehen sein können, die eine Infektionsquelle für die damit in Berührung kommenden Personen darstellen können. Krankenhausabfälle werden oftmals vor ihrem Abtransport zu einer Entsorgungsanlage zerkleinert (geschreddert). Auch dieses geschredderte Material kann während der Lagerung und/oder des Transportes erfindungsgemäß behandelt werden, wodurch das infektiöse Gefahrenrisiko, das mit einer Keimbelastung des Materials einhergeht, verringert wird.

Das erfindungsgemäße Verfahren ist aber nicht auf die Behandlung von Abfällen beschränkt. Vielmehr kann das Verfahren auch zur Verbesserung der hygienischen Verhältnisse bei der Lagerung und insbesondere Zwischenlagerung von anderen Materialien eingesetzt werden, beispielsweise bei der Lagerung von frischem Gemüse und/oder frischem Obst. Obst und Gemüse ist oftmals schnell verderblich, wenn es nicht in geeigneter Weise gelagert wird. Mit dem erfindungsgemäßen Verfahren kann die Frischhaltung von Obst und Gemüse erheblich verbessert werden, indem das Obst und/oder Gemüse in einem Behälter zwischengelagert wird, in den ein mit Ozonid oder einer Mischung aus Ozonid und Hydroperoxid versetzter Träger eingebracht wird oder an dem ein solcher Träger angebracht wird. Durch die sich entwickelnden Gase oder Dämpfe im Zusammenspiel mit dem von dem Obst und/oder Gemüse abgesonderten Wasserdampf entwickelt sich eine desinfizierende Atmosphäre, die die welkenden und verderbenden Prozesse bei dem Obst oder Gemüse deutlich verlangsamt.

In einer weiteren Ausgestaltung des erfindungsgemäßen Verfahrens handelt es sich bei dem Material um kultivierbare Pflanzen, die im Hinblick auf eine Schädlingsbekämpfung behandelt werden. Versuche des Erfinders haben gezeigt, dass durch den Einsatz eines mit organischen Ozoniden oder einer Mischung aus organischen Ozoniden und organischen Hydroperoxiden versetzten Trägers eine effektive Schädlingsbekämpfung bei Pflanzen durchgeführt werden kann, wobei die Pflanze selbst sich sehr schnell von dem Schädlingsbefall erholt und sich im Weiteren besonders gut entwickelt. Das erfindungsgemäße Verfahren hat dabei den besonderen Vorteil, dass sehr unterschiedliche Schädlinge wirksam bekämpft werden können, da die verwendeten Ozonide und gegebenenfalls zusätzlich Hydroperoxide unspezifisch auf verschiedene Pilze, Bakterien, Viren oder Schadinsekten wirken, ohne dass die Pflanze selbst dabei angegriffen wird. Darüber hinaus stellt die Behandlung mit Ozoniden und gegebenenfalls zusätzlich Hydroperoxiden keine Belastung für die Umwelt dar, da sich diese Substanzen im Zuge der Anwendung selbst vollständig abbauen und dabei zusätzlich einen reinigenden Effekt insbesondere in der Luft im Sinne einer Luftentkeimung bewirken. Weiterhin üben die verwendeten organischen Ozonide oder die Mischung aus den Ozoniden und den Hydroperoxiden in gewisser Weise eine Depotwirkung aus, die sich über den gesamten Zeitraum des oxidativ wirkenden Abbaus dieser Substanzen erstreckt, sodass die Behandlung mit einer verhältnismäßig geringen Dosis dieser Substanzen erfolgen kann. Da die Wirkung der Ozonide und gegebenenfalls zusätzlich der organischen Hydroperoxide auf einer verhältnismäßig unspezifischen oxidativen Wirkung beruht, können sich Resistenzen der Schadorganismen gegenüber dieser Behandlung nicht entwickeln. Wenn die Ozonide oder die Mischung aus den Ozoniden und den Hydroperoxiden erfindungsgemäß verwendet werden, kann auf andere beispielsweise chemische Behandlungsmethoden (z. B. Spritzen von Insektiziden) verzichtet werden. Somit ergibt sich ein erheblicher Vorteil für die Umwelt, da das erfindungsgemäße Verfahren bei der Pflanzenbehandlung keine Umweltbelastung darstellt. Das Verfahren zur Pflanzenbehandlung kann mit Vorteil in einem geschlossenen Behälter durchgeführt werden, da sich in einem geschlossenen Raum verhältnismäßig hohe Konzentrationen der Ozonid-/Hydroperoxiddämpfe einstellen. Es ist aber auch möglich, die Behandlung der Pflanzen im Freiland durchzuführen, indem beispielsweise erfindungsgemäß benetzte Träger in den Pflanzenbestand eingebracht werden. Beispielsweise können kleine Träger aus Pappe oder saugfähigem Vliesmaterial (z. B. Kärtchen oder Bänder), die mit der Ozonid-/Hydroperoxidsubstanz getränkt sind, in den Pflanzenbestand gehängt oder beispielsweise in den Boden gesteckt werden. Die erfindungsgemäße Wirkung basiert auf den sich entwickelnden Gasen oder Dämpfen, die sich in der Masse der organischen Ozonide und gegebenenfalls zusätzlich der Hydroperoxide entwickeln. Von den Pflanzen selbst wird Wasserdampf freigesetzt, wobei durch Kondensation und/oder Tau und Regen im Pflanzenbestand auch flüssiges Wasser auftritt. Bei Kontakt der gasförmigen Phase der organischen Ozonid-/Hydroperoxidmasse selbst mit dieser Feuchtigkeit bildet sich vor allem Wasserstoffperoxid, welches oxidierend und desinfizierend wirkt. Etwaige Carbonsäuren, die bei diesen Vorgängen als Abbauprodukte ebenfalls gebildet werden, begünstigen die Kondensation und beschleunigen die Prozesse. Die hierbei entstehende oxidierende Atmosphäre verhindert und bekämpft die Entwicklung von Bakterien, Pilzen, Viren und/oder Insekten zuverlässig und entfaltet damit eine schädlingsbekämpfende Wirkung im Pflanzenbestand. Weiterhin ist es möglich, den Pflanzenbestand nach dem Einbringen der erfindungsgemäß verwendet Träger abzudecken, beispielsweise mit Folie, oder einen Pflanzenbestand in einem Gewächshaus oder in einem Zelt zu behandeln.

Weitere Merkmale und Vorteile der Erfindung ergeben sich aus der nachfolgenden Beschreibung von Ausführungsbeispielen in Verbindung mit den Zeichnungen. Hierbei können die einzelnen Merkmale jeweils für sich oder in Kombination miteinander verwirklicht sein.

In den Zeichnungen zeigen:
Figur 1 schematische Darstellung eines Abfallbehälters mit einer erfindungsgemäß verwendbaren Einrichtung;
Figur2 schematische Darstellung einer weiteren Ausführungsform eines Abfallbehälters mit einer erfindungsgemäß verwendbaren Einrichtung;
Figur3 schematische Darstellung eines Behälters mit einer zu behandelnden Pflanze und mit einer erfindungsgemäß verwendbaren Einrichtung;
Figur4 schematische Darstellung einer Behälteranordnung zur Zwischenlagerung von Obst und Gemüse mit einer erfindungsgemäß verwendbaren Einrichtung; und
Figur 5 schematische Darstellung einer weiteren Ausführungsform einer erfindungsgemäß verwendbaren Einrichtung.

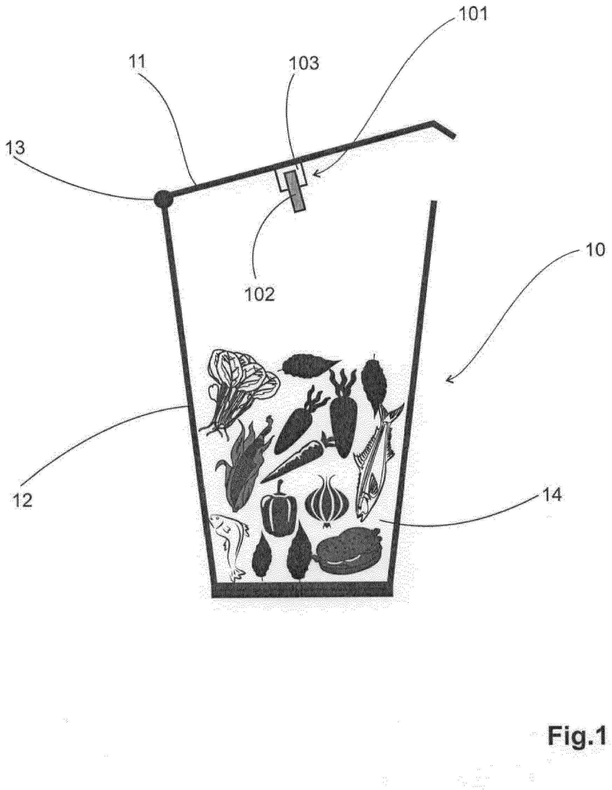

Fig. 1 illustriert einen Abfallbehälter 10, der mit einer Einrichtung 101 ausgestattet ist. Wesentlicher Bestandteil der Einrichtung 101 ist ein Träger 102, der mit einem organischen Ozonid oder einer Mischung aus einem organischen Ozonid und einem organischen Hydroperoxid versetzt ist. Der Träger 102 ist in einer Halterung 103 der Einrichtung 101 eingeklemmt. Die Halterung 103 ist an der Innenseite eines Deckels 11 des Abfallbehälters 10 befestigt. Hierbei ist der Deckel 11 an einem Korpus 12 des Abfallbehälters 10 über ein Scharnier 13 befestigt. Im Inneren des Behälters 10 befinden sich organische Abfälle 14. Der Träger 102 (z. B. Pad) besteht vorzugsweise aus einem porösen, saugfähigen Material, das mit dem Ozonid oder der Mischung aus dem Ozonid und dem Hydroperoxid, welches insbesondere in öliger bis pastöser Form bereitgestellt wird, getränkt oder benetzt ist. Bei geschlossenem Deckel 11 verdampft das Ozonid und gegebenenfalls zusätzlich das Hydroperoxid bzw. geht in die Gasphase über und bildet mit dem im Inneren des Behälters vorhandenen Wasserdampf ein oxidierendes und desinfizierendes Aerosol. In dem Aerosol entwickelt sich Wasserstoffperoxid. Versuche haben gezeigt, dass sich ein Kaliumiodid-Stärkepapier (z. B. Kaliumiodidstreifen der Firma Merck) innerhalb von vier Stunden dunkelviolett verfärbt. Dies stellt einen unspezifischen Nachweis für das Vorhandensein von Oxidationsmitteln im Inneren des Behälters dar. Der pH-Wert im Inneren des Behälters liegt in nachweisbarer Weise unterhalb von 7, da sich Carbonsäuren bilden. Die mit diesem Nachweis allgemein nachweisbaren Oxidationsmittel sind im Wesentlichen auf das sich bildende Wasserstoffperoxid zurückzuführen. Diese Ergebnisse belegen, dass allein durch die entstehenden Dämpfe der Ozonid-/Hydroperoxidmasse in Verbindung mit den Wasserdämpfen sich eine Atmosphäre mit desinfizierender Wirkung im Inneren des Behälters einstellt, sodass ein desinfizierendes Milieu geschaffen wird, welches für eine deutliche Verbesserung der hygienischen Bedingungen in dem Behälter 10 sorgt, sodass ein Keimbefall oder ein Pilzbewuchs sowie auch ein Insektenbefall zuverlässig vermieden oder verringert wird. Die Dämpfe der Ozonid-/Hydroperoxidmasse und die desinfizierende Atmosphäre dringen durch Diffusion selbst in die Materialien im Behälter 10, also in die Abfallbestandteile 14 ein.

Prinzipiell ist es möglich, für den Träger ein glattes Material zu verwenden. Um aber die Aufnahmekapazität und die Haftung der Ozonid-/Hydroperoxidmasse an dem Träger zu verbessern, sind Materialien mit einer vergrößerten und/oder adhäsiven Oberfläche und insbesondere mit einer gewissen Porosität besonders bevorzugt. Geeignete Träger können beispielsweise maschig oder gewebig sein. Besonders geeignet sind vliesartige Gewebe, die zusätzlich auch kompostierbar sind, um sie zusammen mit beispielsweise Bioabfällen entsorgen zu können. Insbesondere im Zusammenhang mit kompostierbaren Abfällen ist es besonders vorteilhaft, wenn der Träger aus einem biologisch abbaubaren Material besteht. Hierdurch ist es möglich, den gebrauchten Träger zusammen mit dem kompostierbaren Müll zu entsorgen. Ein besonderer Vorteil des erfindungsgemäßen Ansatzes in diesem Zusammenhang ist, dass sich auch das Ozonid bzw. die Ozonid-/Hydroperoxidmasse selbst von sich aus abbaut. Das Ozonid zerstört sich gewissermaßen selbst durch das von ihm selber produzierte Wasserstoffperoxid. Die Reaktionsprodukte sind immer Aldehyde, Carbonsäuren und Wasser, bis zuletzt nur noch Kohlendioxid und Wasser übrig bleibt. Das Gleiche gilt für das Hydroperoxid. Dieser Abbauvorgang findet während der Zwischenlagerung der Materialien statt, kann aber auch noch weiter anhalten, während das Material beispielsweise als Bioabfall abtransportiert wird, wodurch die desinfizierende Wirkung auch während des Abtransports anhält. Es entstehen keinerlei Produkte, die eine Kompostierbarkeit des Abfalls behindern würden.

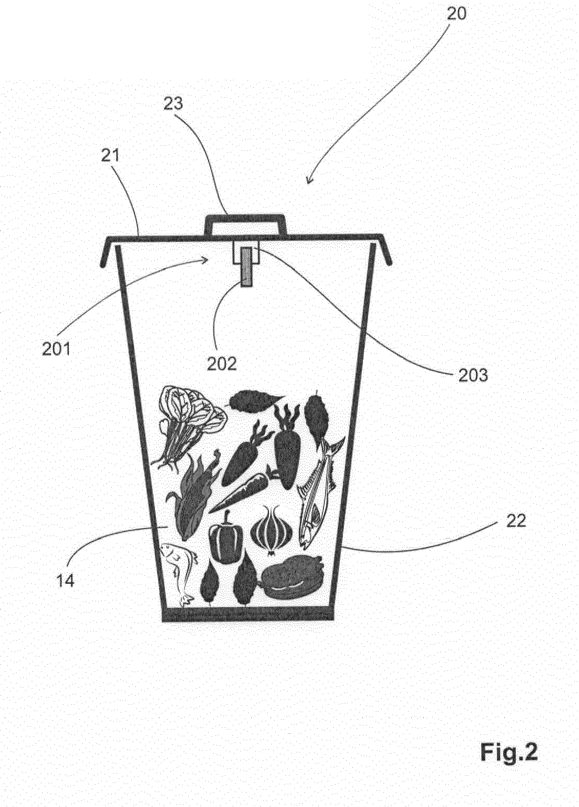

Fig. 2 zeigt ein weiteres Beispiel für einen Abfallbehälter 20 mit einer Einrichtung 201. Der Behälter 20 besteht aus einem Korpus 22 und einem Deckel 21. In diesem Fall handelt es sich um einen losen Deckel 21, der mit einem Griff 23 zum Öffnen und Schließen des Abfallbehälters 20 versehen ist. Vergleichbar mit dem Abfallbehälter 10 in der Fig. 1 ist auch bei dem Abfallbehälter 20, der in Fig. 2 gezeigt ist, auf der Innenseite des Deckels 21 die Einrichtung 201 befestigt, die eine Halterung 203 und einen darin eingesetzten Träger 202, der mit einem organischen Ozonid oder einer Mischung aus einem organischen Ozonid und einem organischen Hydroperoxid versetzt ist, umfasst. Für die Befestigung des Trägers 202 in der Halterung 203 kann beispielsweise eine Klemmung oder Quetschung oder auch eine andere Befestigungsart vorgesehen sein. Beispielsweise kann der Träger 202 mittels eines Klettverschlusses, einer Nadel, einer Klebung oder Ähnlichem an der Halterung 203 befestigt sein. Vorzugsweise ist diese Befestigung reversibel, sodass der Träger 202 nach einer gewissen Anwendungszeit entnommen und ausgetauscht werden kann. In besonders praktischer Weise kann der gebrauchte Träger 202 zusammen mit dem Abfall 14 aus dem Behälter 20 entsorgt werden. Prinzipiell ist es auch möglich, dass die Ozonid- oder Hydroperoxidbeladung des benutzten Trägers 202 erneuert wird, sodass der Träger nicht entsorgt werden muss.

Der Behälter 10 oder 20 ist in besonderer Weise für die Zwischenlagerung von Abfällen, die mit einer Geruchsentwicklung und/oder einer besonderen Keimbelastung einhergehen, geeignet, beispielsweise für Hygieneabfälle (Babywindeln oder andere Hygieneartikel) oder Krankenhausabfälle. In ebenfalls besonders vorteilhafter Weise sind derartige Abfallbehälter für die Zwischenlagerung von Restmüll oder von kompostierbarem Müll (Biomüll) geeignet, da insbesondere bei diesen Abfällen der Anteil von organischem Material sehr hoch ist oder der Abfall sogar vollständig aus organischem Material besteht. Dieses Material ist insbesondere bei längerer Zwischenlagerungsdauer und vor allem auch bei erhöhten Temperaturen besonders anfällig für einen Keimbefall, einen Pilzbewuchs und für einen Insektenbefall. Durch die Einrichtung können die hygienischen Verhältnisse in derartigen Abfallbehältern so verbessert werden, dass derartige Prozesse vermieden oder zumindest deutlich verlangsamt werden, sodass es nicht zu unangenehmen Geruchsbelästigungen oder gar zu gesundheitlichen Risiken durch beispielsweise einen extremen Schimmelbefall oder Insektenbefall kommt.

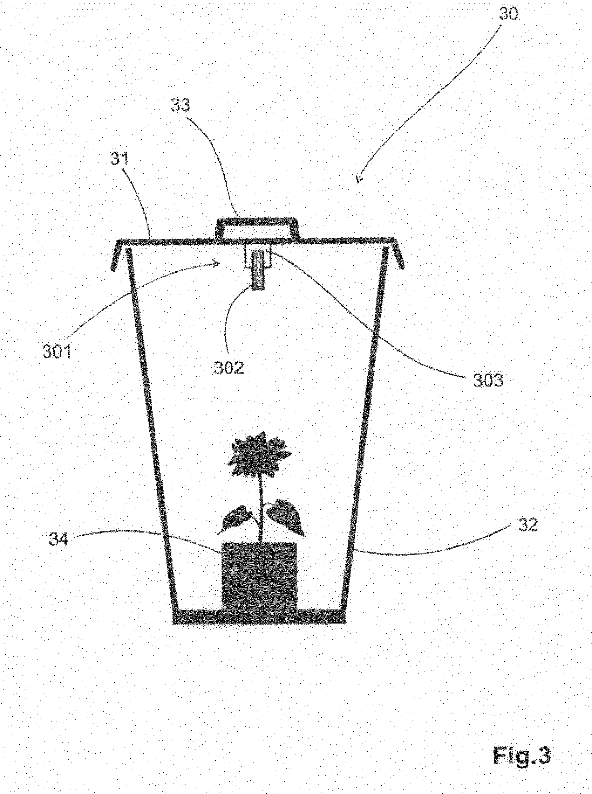

Fig. 3 illustriert einen Behälter 30 zur Aufnahme einer oder mehrerer Pflanzen 34, die erfindungsgemäß mit einem Ozonid/Hydroperoxid-Träger 302 behandelt werden. Der Behälter 30 entspricht im Wesentlichen dem anhand der Fig. 2 erläuterten Behälter 20. In vergleichbarer Weise umfasst der Behälter 30 einen Korpus 32 und einen Deckel 31 mit einem Griff 33. Auf der Innenseite des Deckels 31 ist eine Einrichtung 301 mit einer Halterung 303 für den Träger 302 vorgesehen, wobei der Träger 302 erfindungsgemäß mit einem organischen Ozonid oder einer Mischung aus einem organischen Ozonid und einem organischen Hydroperoxid versetzt ist. Die im Inneren des Behälters entstehenden Ozonid- und Hydroperoxiddämpfe sorgen in Verbindung mit den Wasserdämpfen, die insbesondere auch von der Pflanze 34 ausgehen, für eine oxidative Atmosphäre mit desinfizierender und schädlingsabtötender Wirkung.

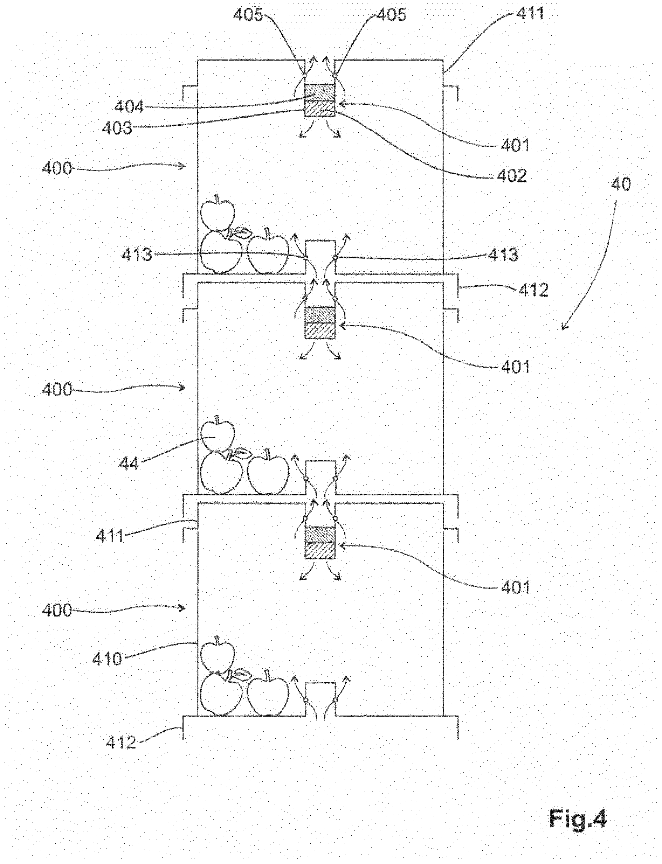

Fig. 4 zeigt ein weiteres Beispiel für einen Behälter 400 bzw. eine Behälteranordnung 40, die mit Einrichtungen 401 ausgestattet ist. Die Behälteranordnung 40 ist zur Zwischenlagerung von frischem Obst 44 oder Gemüse vorgesehen. Die Behälteranordnung 40 wird von mehreren stapelbaren einzelnen Behältern 400 gebildet, die jeweils aus einer Behälterwanne 410 und einem Deckel 411 bestehen. Der Boden der jeweiligen Behälterwanne 410 ist mit einem Stapelrand 412 versehen. In das Innere des jeweiligen Deckels 411 ist eine Vertiefung (z.B. Nut) 403 eingelassen, die nach unten durchlässig und insbesondere gasdurchlässig ist. Innerhalb der Vertiefung 403 befindet sich poröses Trägermaterial 402, das den Träger für das organische Ozonid oder die Mischung aus dem Ozonid und dem organischen Hydroperoxid 404, das oder die beispielsweise als pastöse Masse auf das Trägermaterial 402 aufgebracht ist, bildet. Im oberen Bereich der Vertiefung 403 befinden sich Ventilationsöffnungen 405. Auch im unteren Bereich des Behälters 400 sind in einer Aufstülpung der Bodenplatte der Behälterwanne 410 Ventilationsöffnungen 413 vorgesehen. Durch die unteren und oberen Ventilationsöffnungen 413 und 405 ist es möglich, dass von unten nach oben in Folge einer natürlichen Konvektion eine Luftdurchströmung, angedeutet durch die Pfeile von unten nach oben, durch die einzelnen Behälter 400 hindurch erfolgt. Prinzipiell ist es auch möglich, eine beispielsweise mit einem Ventilator angetriebene Behälterbelüftung vorzusehen. Die Benutzung der einzelnen Behälter 400 kann stapelweise in der hier gezeigten Form oder auch einzeln erfolgen.

Die Funktionsweise der Einrichtung 401 bei dieser Ausgestaltung der Behälter 400 entspricht im Wesentlichen der oben bereits beschriebenen Funktion bei Abfallbehältern. Die Ozonid-/Hydroperoxidmasse 404 dringt in das poröse Trägermaterial 402 ein. Da das organische Ozonid oder die Mischung aus dem organischen Ozonid und dem Hydroperoxid einen gewissen Dampfdruck aufweist, kommt es zur Ausbildung von Dämpfen (Ozonid- und gegebenenfalls zusätzlich Hydroperoxidgase), die über den durchlässigen Bereich, der das Trägermaterial 402 nach unten begrenzt, in das Innere des jeweiligen Behälters 400 eindringen. Die Früchte 44 sondern Wasserdampf ab, sodass es zusammen mit den Dämpfen der Ozonid-/Hydroperoxidmasse zur Ausbildung einer wasserstoffperoxidhaltigen Atmosphäre im Inneren der Behälter 400 kommt. Der pH-Wert des dabei gebildeten sekundären organischen Aerosols liegt beispielsweise bei pH 4,5, wenn beispielsweise ozonisierte Ölsäure als Ozonidmasse 404 eingesetzt wird. Hierbei färbt sich ein Kaliumiodid-Stärke-Teststreifen als Nachweismittel für Oxidationsmittel innerhalb von circa vier Stunden in einem geschlossenen Gefäß mit einem Fassungsvolumen von 1 Liter dunkelviolett. Das gebildete sekundäre organische Aerosol wirkt bakterizid und fungizid. Als zusätzlicher vorteilhafter Effekt insbesondere bei der Zwischenlagerung von Obst oder Gemüse wird das Reifegas Ethylen, das von vielen Früchten während der Lagerung gebildet wird und die Abbauvorgänge in der Frucht beschleunigt, durch die peroxidische Atmosphäre über den Zwischenschritt eines Alkohols bis zur Carbonsäure oxidiert, sodass die Nachreifung aufgehalten und das Obst oder Gemüse länger frisch bleibt.

Das Trägermaterial 402 kann mit der Ozonid-/Hydroperoxidmasse 404 beispielsweise bestrichen oder getränkt werden. Weiterhin kann auf das poröse Trägermaterial 402 auch ein vorab mit der Ozonid-/Hydroperoxidmasse versetzter weiterer Träger, beispielsweise ein mit Ozonid getränktes Vlies, aufgebracht werden. Der weitere Träger kann beispielsweise auch mit einer Klebung versehen sein und auf der Innenseite des Deckels 411 oder der Behälterwanne 410 angeklebt werden. In diesem Fall kann auf das poröse Trägermaterial (Trägerschicht) 402 in der Vertiefung 403 verzichtet werden. Prinzipiell ist es auch möglich, einen mit Ozonid oder einer Mischung aus Ozonid und Hydroperoxid versetzten Träger von außen an den Deckel 411 oder an die Außenseite der Behälterwanne 410 aufzukleben, wenn in der Wandung des Behälters 400 an der entsprechenden Stelle eine Perforation vorgesehen ist, damit die sich entwickelnden Dämpfe in das Innere des Behälters 400 eindringen können.

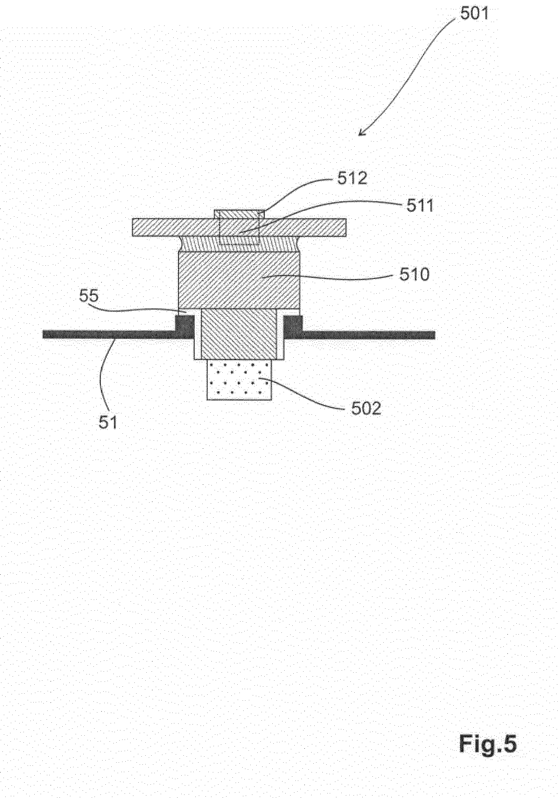

Fig. 5 illustriert ein weiteres Beispiel für eine Einrichtung 501, die in Form eines Steckknopfes mit einem integrierten Vorratstank 510 für die Ozonid-/Hydroperoxidmasse ausgestaltet ist. Der Vorratstank 510 kann über eine Füllöffnung 511 befüllt werden, wobei die Füllöffnung 511 mit einem Deckel oder einer Kappe 512 verschließbar ist. Die steckknopfförmige Einrichtung 501 bildet zugleich eine Halterung für den Träger 502, der beispielsweise aus einem Vliesmaterial besteht. Der Träger 502 steht in direktem Kontakt mit der Ozonid-/Hydroperoxidmasse im Vorratstank 510 und saugt sich dabei ständig mit frischem Ozonid/Hydroperoxid voll. Die Einrichtung 501 ist in eine entsprechende Öffnung eines Deckels 51 eines hier nicht näher dargestellten Behälters eingesteckt, sodass der Träger 502 in das Innere des Behälters hineinragt. Die Einrichtung 501 kann dabei mittels einer Dichtung 55, z. B. einem Gummiring, gegenüber der Öffnung im Deckel 51 abgedichtet sein. Diese Ausgestaltung der Einrichtung 501 mit dem Vorratstank 510 hat den besonderen Vorteil, dass die Einrichtung 501 für einen längeren Zeitraum angewendet werden kann, da der Träger 502 ständig mit frischem Ozonid/Hydroperoxid versorgt wird. Hierbei kann gegebenenfalls der Vorratstank 510 wiederholt mit frischer Ozonid-/Hydroperoxidmasse vom Anwender befüllt werden, sodass die Einrichtung 501 im Prinzip für einen unbegrenzten Zeitraum verwendet werden kann.

Für die Herstellung der organischen Ozonide oder der Mischung aus den organischen Ozoniden und den organischen Hydroperoxiden werden ungesättigte Fettsäuren mit ozonhaltigem und vorzugsweise gasförmigem Sauerstoff behandelt. In besonders bevorzugter Weise werden hierfür Pflanzenöle eingesetzt. Beispielsweise ist Ölsäure besonders geeignet, wobei die Ölsäure aus einer pflanzlichen oder auch aus einer tierischen Quelle stammen kann. Die Ozonisierung der Fettsäure erfolgt vorzugsweise mit trockenem sauerstoffhaltigem Gas, vorzugsweise mit trockenem medizinischem oder industriellem Sauerstoff in einer Reinheit von > 99,5 oder > 99,9% (wasserfrei), der mit Ozon angereichert wurde. Die "Trockenheit" des Sauerstoffs lässt sich über den Taupunkt definieren. Beispielsweise ist "trockener" Sauerstoff mit einem Taupunkt im Bereich von 200 Kelvin für die erfindungsgemä-ßen Zwecke geeignet. Der Ozonisierungsprozess wird vorzugsweise in einem geschlossenen Gefäß mit Kühlung durchgeführt, wobei in dem Gefäß die zu ozonisierende Fettsäure vorgelegt wird und bei intensiver Durchmischung der ozonhaltige trockene Sauerstoff eingeleitet wird. Vorteilhafterweise wird der Prozess unter vollständigem Ausschluss von Wasser durchgeführt, wobei zweckmäßigerweise wasserdampfundurchlässige Gefäße, Leitungen usw. (diffusionsdicht für gasförmiges Wasser) verwendet werden. Der Prozess kann beispielsweise im Batch-Verfahren bei einer Ozonisierungszeit von etwa 4 h durchgeführt, werden. Dabei bildet sich an der C-C-Doppelbindung der Fettsäure ein Ozonid. Ein weiteres Reaktionsprodukt kann sich in Gegenwart von Wasser mit einer funktionellen HOO-Peroxidgruppe am α-ständigen C-Atom bilden. Quelle für das Wasser kann beispielsweise das zugeführte gegebenenfalls feuchte, mit Ozon angereicherte Sauerstoffgas sein oder die Umgebungsatmosphäre beim Ozonisieren der Fettsäure oder eine gegebenenfalls vorgesehene Wasserzugabe zu der Fettsäure bzw. zu dem Reaktionsansatz vor dem Beginn der Ozonisierung. Je nach Herstellungsbedingungen bildet sich ein öliges bis pastöses Produkt. Dieses Addukt ist beständig und lagerfähig. Der Dampfdruck steigt mit dem Grad der Ozonisierung und ist von den Ausgangssubstanzen und den Reaktionsbedingungen abhängig. Für die Fettsäure-Komponente bei der Ozonid- /Hydroperoxidherstellung können prinzipiell auch Mischungen verschiedener Fettsäuren und insbesondere Mischungen verschiedener Pflanzenöle, beispielsweise verschiedene ungesättigte Triglyceride, gegebenenfalls mit weiteren Zusätzen, verwendet werden.

Besonders bevorzugtes Ausgangsprodukt für die Herstellung der organischen Ozonide ist Ölsäure. Die Ölsäure kann beispielsweise aus Olivenöl stammen, das zu etwa 70 % aus Ölsäure besteht. Vorzugsweise erfolgt die Ozonisierung der reinen Ölsäure mit trockenem, ozonhaltigen Sauerstoff unter aprotischen Bedingungen. Als Produkt dieses Prozesses wird eine ölige bis pastöse Ozonidmasse erhalten, die frei von störenden Nebenprodukten, wie z.B. Aldehyde, Ketone und Wasserstoffperoxid, ist. Die Ozonidmasse ist stabil und über einen langen Zeitraum lagerfähig. Für eine Stabilisierung dieser Ozonidmasse sind keine weiteren Zusätze, keine Extraktion und keine Reduktionsmittel erforderlich. Der Dampfdruck der ozonisierten Ölsäure kann durch den Grad der Ozonisierung angepasst werden. Der Dampfdruck ist bei höherer POZ größer. Weiterhin zeichnet sich eine Ozonidmasse mit einer höheren POZ im Allgemeinen durch einen stärkeren Geruch aus. Prinzipiell ist für größere Behälter ein höherer Dampfdruck des organischen Ozonids vorteilhaft. Auch die Konsistenz der Ozonidmasse wird durch den Grad der Ozonisierung beeinflusst. Für die meisten Anwendungen eignet sich insbesondere eine pastöse Masse.

## Patentansprüche

1. Verwendung einer zur Anbringung in einem Behälter (10; 20; 30; 400) vorgesehenen Einrichtung (101; 201; 301; 401; 501), zur Bereitstellung einer Atmosphäre mit desinfizierender Wirkung in dem Behälter, wobei der Behälter für eine Zwischenlagerung von Material (14; 34; 44) vorgesehen ist und die Einrichtung wenigstens einen Träger (102; 202; 302; 402; 502) umfasst, der mit organischen Ozoniden oder einer Mischung aus organischen Ozoniden und organischen Hydroperoxiden (404) versetzt ist, die durch eine Behandlung von ungesättigten Fettsäuren mit ozonhaltigem Sauerstoff hergestellt wurden.

2. Verwendung nach Anspruch 1, **dadurch gekennzeichnet, dass** die organischen Ozonide (404) durch eine Behandlung von ungesättigten Fettsäuren, insbesondere Ölsäure, mit trockenem ozon haltigen Sauerstoff herstellbar sind.

3. Verwendung nach einem der vorhergehenden Ansprüche, **dadurch gekennzeichnet, dass** die organischen Ozonide und/oder organischen Hydroperoxide eine Peroxidzahl (POZ) in einem Bereich zwischen etwa 50 - 5000 meq/kg, vorzugsweise zwischen etwa 100 - 2500 meq/kg, aufweisen.

4. Verwendung nach einem der vorhergehenden Ansprüche, **dadurch gekennzeichnet, dass** die Einrichtung (101; 201; 301; 401; 501) eine Halterung (103; 203; 303; 403) für den Träger (102; 202; 302; 402; 502) umfasst, wobei die Halterung im inneren Bereich des Behälters (10; 20; 30; 400) anbringbar ist, wobei vorzugsweise die Halterung (103; 203; 303; 403) an der Innenseite eines Deckels (11; 21;31; 411) des Behälters anbringbar ist.

5. Verwendung nach einem der vorhergehenden Ansprüche, **dadurch gekennzeichnet, dass** der Träger (102; 202; 302; 402; 502) austauschbar ist.

6. Verwendung nach einem der vorhergehenden Ansprüche, **dadurch gekennzeichnet, dass** der Träger (102; 202; 302; 402; 502) ein poröses Material und/oder zumindest teilweise eine adhäsive Oberfläche umfasst.

7. Verwendung nach einem der vorhergehenden Ansprüche, **dadurch gekennzeichnet, dass** der Träger (102; 202; 302; 402; 502) im Wesentlichen aus einem biologisch abbaubaren Material besteht.

8. Verwendung (501) nach einem der vorhergehenden Ansprüche, **dadurch gekennzeichnet, dass** die Einrichtung einen Vorratstank (510), insbesondere einen wiederbefüllbaren Vorratstank, für die organischen Ozonide und/oder organischen Hydroperoxide umfasst.

9. Verwendung eines Trägers (102; 202; 302; 402; 502), der mit organischen Ozoniden und/oder organischen Hydroperoxiden (404) versetzt ist, die durch eine Behandlung von ungesättigten Fettsäuren mit ozonhaltigem Sauerstoff hergestellt wurden, zur Bereitstellung einer Atmosphäre mit desinfizierender Wirkung in einem Behälter, der für eine Zwischenlagerung von Material vorgesehen ist.

10. Verfahren zur Behandlung von Material (14; 34; 44), **dadurch gekennzeichnet, dass** das Material in einem Behälter (10; 20; 30; 400) zwischengelagert wird, wobei in den Behälter ein mit organischen Ozoniden oder einer Mischung aus organischen Ozoniden und organischen Hydroperoxiden versetzter Träger (102; 202; 302; 402; 502) eingebracht wird und eine desinfizierende Wirkung durch in das Innere des Behälters (10; 20; 30; 400) entweichende Ozonid- und/oder Hydroperoxiddämpfe erzielt wird, wobei die organischen Ozonide oder die Mischung aus den organischen Ozoniden und den organischen Hydroperoxiden durch eine Behandlung von ungesättigten Fettsäuren mit ozonhaltigem Sauerstoff hergestellt wurden.

11. Verfahren nach Anspruch 10, **dadurch gekennzeichnet, dass** das Material organische Abfälle (14) oder Hygieneabfälle oder Krankenhausabfälle umfasst.

12. Verfahren nach Anspruch 10, **dadurch gekennzeichnet, dass** das Material frisches Gemüse und/oder frisches Obst (44) umfasst.

13. Verfahren nach Anspruch 10, **dadurch gekennzeichnet, dass** das Material kultivierbare Pflanzen (34) umfasst.

14. Mit einem Deckel versehener, für eine Zwischenlagerung von Material vorgesehener, verschließbarer Behälter, umfassend einen Träger,
• der mit organischen Ozoniden oder einer Mischung aus organischen Ozoniden und organischen Hydroperoxiden versetzt ist, die durch eine Behandlung von ungesättigten Fettsäuren mit ozonhaltigem Sauerstoff hergestellt wurden, und
• der in dem Behälter eine Atmosphäre mit desinfizierender Wirkung bereitstellt.

## Claims

1. Use of a device (101; 201; 301; 401; 501), intended for mounting in a vessel (10; 20; 30; 400), for provision of an atmosphere having disinfecting action in the vessel, wherein the vessel is intended for intermediate storage of material (14; 34; 44) and the device comprises at least one carrier (102; 202; 302; 402; 502) that has been admixed with organic ozonides or a mixture of organic ozonides and organic hydroperoxides (404) that have been produced by a treatment of unsaturated fatty acids with ozone-containing oxygen.

2. Use according to Claim 1, **characterized in that** the organic ozonides (404) are preparable by a treatment of unsaturated fatty acids, especially oleic acid, with dry ozone-containing oxygen.

3. Use according to either of the preceding claims, **characterized in that** the organic ozonides and/or organic hydroperoxides have a peroxide number (PON) within a range between about 50-5000 meq/kg, preferably between about 100-2500 meq/kg.

4. Use according to any of the preceding claims, **characterized in that** the device (101; 201; 301; 401; 501) has a holder (103; 203; 303; 403) for the carrier (102; 202; 302; 402; 502), where the holder can be mounted in the interior of the vessel (10; 20; 30; 400), where the holder (103; 203; 303; 403) can preferably be mounted on the inside of a lid (11; 21; 31; 411) of the vessel.

5. Use according to any of the preceding claims, **characterized in that** the carrier (102; 202; 302; 402; 502) is exchangeable.

6. Use according to any of the preceding claims, **characterized in that** the carrier (102; 202; 302; 402; 502) comprises a porous material and/or, at least to some degree, an adhesive surface.

7. Use according to any of the preceding claims, **characterized in that** the carrier (102; 202; 302; 402; 502) consists essentially of a biodegradable material.

8. Use (501) according to any of the preceding claims, **characterized in that** the device comprises a reservoir tank (510), especially a refillable reservoir tank, for the organic ozonides and/or organic hydroperoxides.

9. Use of a carrier (102; 202; 302; 402; 502) admixed with organic ozonides and/or organic hydroperoxides (404) which has been produced by a treatment of unsaturated fatty acids with ozone-containing oxygen for provision of an atmosphere having disinfecting action in a vessel intended for intermediate storage of material.

10. Method of treatment of material (14; 34; 44), **characterized in that** the material is being stored intermediately in a vessel (10; 20; 30; 400), wherein a carrier (102; 202; 302; 402; 502) admixed with organic ozonides and/or a mixture of organic ozonides and organic hydroperoxides is introduced in the vessel and a disinfecting action is achieved by ozonide and/or hydroperoxide vapours that escape into the interior of the vessel (10; 20; 30; 400), wherein the organic ozonides or the mixture of the organic ozonides and the organic hydroperoxides has been produced by a treatment of unsaturated fatty acids with ozone-containing oxygen.

11. Method according to Claim 10, **characterized in that** the material comprises organic wastes (14) or hygiene wastes or hospital wastes.

12. Method according to Claim 10, **characterized in that** the material comprises fresh vegetables and/or fresh fruit (44).

13. Method according to Claim 10, **characterized in that** the material comprises cultivable plants (34).

14. Closable vessel that has been provided with a lid and is intended for intermediate storage of material, comprising a carrier
• that has been admixed with organic ozonides or a mixture of organic ozonides and organic hydroperoxides that have been produced by a treatment of unsaturated fatty acids with ozone-containing oxygen, and
• that provides an atmosphere having disinfecting action in the vessel.

## Revendications

1. Utilisation d'un dispositif (101 ; 201 ; 301 ; 401; 501) prévu pour une mise en place dans un récipient (10, 20; 30; 400), pour réaliser une atmosphère ayant un effet désinfectant dans le récipient, le récipient étant prévu pour un stockage intermédiaire d'un matériau (14; 34; 44), et le dispositif comprenant au moins un support (102 ; 202 ; 302; 402; 502), qui est additionné d'ozonures organiques ou d'un mélange d'ozonures organiques et d'hydroperoxydes organiques (404), qui ont été préparés par un traitement d'acides gras insaturés avec de l'oxygène contenant de l'ozone.

2. Utilisation selon la revendication 1, **caractérisée en ce que** les ozonures organiques (404) peuvent être préparés par un traitement d'acides gras inorganiques, en particulier l'acide oléique, avec de l'oxygène sec contenant de l'ozone.

3. Utilisation selon l'une des revendications précédentes **caractérisée en ce que** les ozonures organiques et les hydroperoxydes organiques présentent un indice de peroxyde (IPO) dans une plage comprise entre environ 50 et 5000 méq/kg, de préférence entre environ 100 et 2500 méq/kg.

4. Utilisation selon l'une des revendications précédentes, **caractérisée en ce que** le dispositif (101 ; 201 ; 301 ; 401; 501) comprend une fixation (103; 203; 303; 403) pour le support(102 ; 202 ; 302; 402; 502), la fixation pouvant être mise en place dans un domaine intérieur du récipient (10 ; 20 ; 30 ; 400), de préférence la fixation (103 ; 203 ; 303 ; 403) pouvant être mise en place contre la face intérieure d'un couvercle (11 ; 21 ; 31 ; 411).

5. Utilisation selon l'une des revendications précédentes, **caractérisée en ce que** le support (102 ; 202 ; 302 ; 402 ; 502) est remplaçable.

6. Utilisation selon l'une des revendications précédentes, **caractérisée en ce que** le support (102 ; 202; 302; 402; 502) comprend un matériau poreux et/ou au moins partiellement une surface adhésive.

7. Utilisation selon l'une des revendications précédentes, **caractérisée en ce que** le support (102 ; 202; 302; 402; 502) est pour l'essentiel constitué d'un matériau biodégradable.

8. Utilisation selon (501) selon l'une des revendications précédentes, **caractérisée en ce que** le dispositif comprend un réservoir (510), de préférence un réservoir re-remplissable, qui comprend les ozonures organiques et/ou les hydroperoxydes organiques.

9. Utilisation d'un support (102 ; 202 ; 302 ; 402 ; 502), qui est additionné d'ozonures organiques et/ou d'hydroperoxydes organiques (404), qui ont été préparés par un traitement d'acides gras insaturés avec de l'oxygène contenant de l'ozone, pour la réalisation d'une atmosphère ayant un effet désinfectant dans un récipient qui est prévu pour un stockage intermédiaire d'un matériau.

10. Procédé pour le traitement d'un matériau (14 ; 34 ; 44), **caractérisé en ce que** le matériau est placé pour stockage intermédiaire dans un récipient (10 ; 20; 30; 400), un support(102 ; 202; 302; 402 ; 502), additionné d'ozonures organiques ou d'un mélange d'ozonures organiques et d'hydroperoxydes organiques, étant introduit dans le récipient, avec obtention d'un effet désinfectant sous l'effet des vapeurs d'ozonures et/ou d'hydroperoxydes qui se dégagent dans l'intérieur du récipient (10; 20; 30; 400), les ozonures organiques ou le mélange des ozonures organiques et des hydroperoxydes organiques ayant été préparé par un traitement d'acides gras insaturés avec de l'oxygène contenant de l'ozone.

11. Procédé selon la revendication 10, **caractérisé en ce que** le matériau comprend des déchets organiques (14) ou des déchets hygiéniques ou des déchets hospitaliers.

12. Procédé selon la revendication 10, **caractérisé en ce que** le matériau comprend des légumes frais et/ou des fruits frais (44).

13. Procédé selon la revendication 10, dans lequel le matériau comprend des plantes cultivables (34).

14. Récipient pouvant être fermé, pourvu d'un couvercle, prévu pour un stockage intermédiaire d'un matériau, comprenant un support
- qui est additionné d'ozonures organiques ou d'un mélange d'ozonures organiques et d'hydroperoxydes organiques, qui ont été préparés par un traitement d'acides gras insaturés avec de l'oxygène contenant de l'ozone, et
- qui réalise dans le récipient une atmosphère ayant un effet désinfectant.
